# EUROPEAN PATENT SPECIFICATION

(11) **EP 3 270 990 B1**
(45) Date of publication and mention of the grant of the patent: **01.12.2021**
(21) Application number: 16712002.1
(22) Date of filing: 16.03.2016
(51) Int. Cl.: A61M 5/32

(54) **HINGED SHIELD ASSEMBLIES AND RELATED METHODS**
SCHARNIERBLENDENANORDNUNG UND ZUGEHÖRIGE VERFAHREN
ENSEMBLES DE PROTECTION ARTICULÉS ET PROCÉDÉS CORRESPONDANTS

(30) Priority: 19.03.2015 US 201514662678
(43) Date of publication of application: 24.01.2018
(60) Divisional application: 21205316.9
(73) Proprietor: B. Braun Melsungen AG, 34212 Melsungen (DE)
(72) Inventor: BUBENIK, Janko, 34212 Melsungen (DE); DICKEL, Markus, 34212 Melsungen (DE)
(74) Representative: Kinkeldey, Daniela
(86) International application number: PCT/EP2016/055715
(87) International publication number: WO 2016/146701

(56) References cited:
- EP-A1- 1 380 315
- WO-A1-2008/076459
- WO-A2-2010/059345
- WO-A2-2012/071400
- JP-A- H11 299 692
- JP-A- 2002 102 344
- US-A- 5 885 249
- US-A1- 2008 208 138

## Description

### FIELD

Shields for needle devices are generally discussed herein with hinged shield devices for use with hypodermic needles more particularly discussed.

### BACKGROUND

Recapping is a common procedure for periods between drawing up fluids into a syringe and administering injections through a needle. The recapping procedure can occasionally cause needlesticks since users sometime misalign the needles with the openings on the caps. Injuries can also occur after an injection and prior to the discarding of the needles. Needlesticks can be painful, but can also cause great inconvenience because all needlesticks must be reported. Also, since needles related to needlesticks must be discarded, medications contained within the syringes are unnecessarily wasted. Furthermore, fluids linked to these "clean" type of needlesticks can cause injuries and adverse reactions.

Additionally, needles may be damaged by over-rotation of caps during recapping. A damaged needle may be shifted from its normal orientation, causing the tip to stick out from under the cap. This leads to the same dangers of needlesticks outlined above.

WO 2012/071400 A2 relates to shields for needle devices, in particular, hinged shield assemblies including a shield that is pivotably secured to a needle hub.

US 2008/0208138 A1 relates to syringe assemblies including a syringe barrel having an elongate body, a tip on its distal end surrounded by a collar. An elongate needle shield is hingedly connected to the collar. The needle shield is capable of pivoting from an open position wherein the needle cannula is exposed, to a closed needle protecting position wherein the distal end of the needle cannula is within longitudinal opening of the shield. WO 2008/076459 A1 relates to hinged cap devices for use with a syringe including a base defining an interior cavity for mounting onto a tip and a cap connected to the base by a living hinge.

EP 1 380 315 A1 relates to safety shield assemblies having a collar for connecting the shield to a fluid handling device, whereby the shield may be pivoted with respect to the collar. JP HI 1 299692 relates to a toiled lid device and discloses a main body part 1 incorporating a nozzle for washing the private part is constituted of a case cover 1-a and a case plate 1-b. In a toilet lid 2 and a toilet seat 3, a soft closing unit 5 is mounted on a hinge part and is inserted into a groove for coupling the case cover 1-a via a hinge support 4 into which an output shaft 5-a is fitted.

JP 2002-102344 relates a rotation preventing means, which is composed of a projecting part 31 of a protector 3 and a rib 23 of a hub 22 for regulating the axial rotation of the protector 2 to a needle body.

US 5 885 249 A relates to a syringe with a cap, and more specifically, to a syringe with a cap capable of securely locking the cap at an open position and a closed position.

WO 2010/059345 A2 relates to a hinged cap device for use with a syringe comprising a needle hub comprising a cavity for fitting onto a syringe tip, said needle hub comprising a needle comprising a needle shaft and a needle tip; a cap defining a channel for capturing the needle to shield the needle tip; a living hinge connecting the cap to the needle hub; a depressable button lock for locking the needle so that the needle is not displaceable from the cap; said depressable button lock being located on a proximal end of a first side wall of said cap and comprising a pin projecting from said first side wall towards a second side wall of said cap; and a lock receiving plate located on said second side wall of said cap at a location for receiving said pin.

### SUMMARY

Claim 1 defines a hinged shield assembly and claim 12 the method of manufacturing said assembly. The dependent claims define beneficial modifications. The present device comprises a needle shield hingedly connected to a needle hub, such as by way of a ball-and-socket hinge. At least one stop piece is positioned on the needle hub to abut the shield to then stop the shield from over rotating along one rotational directional when the shield is in a closed position over a needle attached to the needle hub. The needle hub can include two stop pieces located on a flange to stop the shield from over rotating. The stop pieces can each include a base or a combination base and a capture element to define a retention space. The two capture elements can be configured to prevent the first and second sidewalls of the shield from spreading or expanding radially of the lengthwise axis of the needle beyond the width of the two stop pieces.

The hinged shield assembly according to the present invention comprises: a needle hub including a including a flange and a first hinge part formed with the flange, a needle extending from the needle hub and comprising a lengthwise axis; a shield including a second hinge part that engages the first hinge part to pivotably secure the shield to the hub, the shield further including a plurality of sidewalls that partially surround the needle when the shield is in a protected position; wherein each of the sidewalls comprises a base end; the hinged shield assembly further comprises at least one stop piece extending radially of the lengthwise axis from the flange of the needle hub and located in a rotational path of the shield such that at least one of the plurality of sidewalls contacts the at least one stop piece to prevent over rotation of the base end of each of the sidewalls beyond an at rest point upon contact with the at least one stop piece in one rotational direction when the shield is in the protected position, and wherein the at least one stop piece includes a flat distal side configured to contact a proximal edge or contact edge of the at least one of the plurality of sidewalls of the shield .

The hinged shield assembly, wherein the at least one stop piece can comprise a base having a radially extending planar surface to abut the shield.

The hinged shield assembly can further comprise a capture element extending from the base in an axial direction to define a retention space.

The hinged shield assembly can further comprise a reduced depth portion formed on at least one of the plurality of sidewalls on a side of the hinged shield assembly corresponding to the at least one stop piece.

The hinged shield assembly can further comprise at least one tab formed on at least one of the plurality of sidewalls for engaging the needle hub in the protected position.

The hinged shield assembly can further comprise at least one notch formed on the needle hub for receiving the at least one tab in the protected position.

The hinged shield assembly wherein one of the plurality of sidewalls can be a central sidewall and wherein a hook can be formed with the central sidewall. The hook can hook onto the needle or a cylinder post holding the needle in a protected position.

The hinged shield assembly wherein the first hinge part can comprise sockets.

The hinged shield assembly wherein the second hinge part can comprise balls.

The hinged shield assembly can further comprise a second stop piece comprising a base and wherein two bases on the needle hub can define a stop width.

The hinged shield assembly wherein a first sidewall and a second sidewall of the plurality of sidewalls can define a shield width and wherein the stop width can wider than the shield width, equal to the shield width, or smaller than the shield width.

The hinged shield assembly, wherein the first sidewall and the central wall can include a slit to enable a base end of the first sidewall to defect relative to the central wall.

The hinged shield assembly, wherein the second sidewall and the central wall can include a slit to enable a base end of the second sidewall to defect relative to the central wall.

The hinged shield assembly wherein the at least one stop piece can be located on a transverse base defining a flange, which can intersect an elongated portion on the needle hub between a distal cylindrical portion and a proximal cylindrical portion.

The hinged shield assembly wherein the flange can include a first edge, a second edge, and a first side edge and wherein the at least one stop piece can be located on the side edge, between the first edge and the second edge.

The hinged shield assembly wherein flange can include an upper surface facing the distal cylindrical portion, and wherein the radially extending planar surface of the base can be offset from the upper surface.

The hinged shield assembly can further comprise a second stop piece having a base with a radially extending planar surface to abut the shield located on a second side of the flange.

The hinged shield assembly wherein the flange can include an upper surface facing the distal cylindrical portion, and wherein the radially extending planar surfaces of the two bases can be offset from the upper surface.

The hinged shield assembly, wherein the flange can include an upper surface facing the distal cylindrical portion, and wherein the radially extending planar surfaces of the two bases can be co-planar with the upper surface.

The hinged shield assembly wherein a notch can be located at the first edge and the first hinge part can be located at the second edge.

The hinged shield assembly can further comprise a plurality of fins formed on the distal cylindrical portion.

The hinged shield assembly wherein the first hinge part can comprise two sockets each with a perimeter defining an opening having an opening diameter.

The hinged shield assembly wherein the second hinge part can comprise a pair of balls fitted into the two sockets.

The hinged shield assembly can further comprise a capture element extending from each of the base.

The hinged shield assembly wherein the two bases can define radially extending members and the capture elements can define axially extending members.

The hinged shield assembly wherein each pair of base and capture element 198 can form a generally "L" shape structure when viewing from an end.

The hinged shield assembly wherein the capture elements can contact a ledge on a reduced depth portion on a respective sidewall.

The hinged shield assembly wherein each stop piece can include a base and a capture element extending from the base in an axial direction to define a retention space.

The hinged shield assembly wherein at least a portion of the base end of the first sidewall and at least a portion of the base end of the second sidewall can both be located in the two retention spaces.

The hinged shield assembly wherein the base end of the first sidewall and the base end of the second sidewall can each comprise a reduced depth portion and wherein the reduced depth portion can be located in a respective retention space.

The hinged shield assembly can further comprise a tab on each of the first and second sidewalls for engaging the needle hub in the protected position.

The hinged shield assembly can further comprise two notches formed on the needle hub for receiving the two tabs in the protected position.

The hinged shield assembly wherein the two stop pieces can define a stop width.

The hinged shield assembly wherein the first sidewall and the second sidewall of the plurality of sidewalls can define a shield width and wherein the stop width can be wider than the shield width, equal to the shield width, or less than the shield width.

The hinged shield assembly wherein the flange can include a first edge, a second edge, a first side edge, a second side edge, and wherein the two stop pieces can be located, one each, on the first side edge and the second side edge between the first edge and the second edge.

A further aspect of the present invention includes a method of manufacturing a hinge shield assembly. The method can comprise: forming a needle hub including a flange and a first hinge part formed with the flange; extending a needle from the needle hub, said needle comprising a lengthwise axis; forming a shield including a second hinge part that engages the first hinge part to pivotably secure the shield to the hub, the shield further including a plurality of sidewalls that partially surround the needle when the shield is in a protected position; wherein each of the sidewalls comprises a base end; and forming two stop pieces extending from the flange radially of the lengthwise axis and positioning the two stop pieces in a rotational path of the shield such that a first sidewall and a second sidewall of the plurality of walls contact the two stop pieces to prevent over rotation of the base end of the first and second sidewalls beyond an at rest point upon contact with the two stop pieces in one rotational direction when the shield is in the protected position.

A further aspect of the present disclosure includes a method of using a hinge shield assembly described elsewhere herein.

Yet another aspect of the present disclosure can include a hinge shield assembly comprising a needle hub having two sockets or two spaced apart balls and a shield having at least three walls defining an interior space for covering a needle, and the other one of the two sockets or two spaced apart balls located on the shield; and wherein two stop pieces are formed with the needle hub and arranged on the needle hub to stop two of the sidewalls from rotating along a rotational direction when the shield moves over the needle in a protective position and wherein the two spaced apart balls are located in the two socket.

The hinged shield assembly can include a shield for shielding a needle extending from a needle hub.

The needle can be secured to a central post or cylinder post formed with the needle hub.

The needle hub may not include a needle but may include instead a male Luer where the central post is located for receiving a separately formed female Luer hub having a needle attached to the separately formed needle hub.

The needle hub can have a female Luer with external threads for receiving a male Luer.

The needle hub can have a threaded collar.

The shield can be pivotable about the hub to expose the needle for performing a medical procedure and to cover the needle following use.

The shield can be configured to be manipulated from a packaged position to a ready-to-use position in which the needle is exposed, from the ready-to-use position to an open position in which the shield is rotated clear of the needle for use, and from the open position to a secured position or protected position in which the shield is rotated to cover the needle and locks to the needle and/or to the needle hub to prevent or deter re-exposing the needle after it has been used.

The hinged shield assembly includes a conventional blister pack for packaging the assembly therein.

The hinged shield assembly can include a removable cap for capping the sharp distal tip or needle tip of the needle prior to use and for packaging.

The removable cap can be a tapered removable cylindrical cap, which can fit over the distal cylindrical portion of the needle hub in a friction fit.

The cap can be sufficiently long to accommodate the size and length of the needle.

An outer diameter of the cap can be sized so that the width of the shield can only extend around part of the removable cap, enabling the base end of the rear wall of the shield to be spaced from the cap and the shield capable of pivoting towards the cap, in which the shield partly covers the cap. Each sidewall can have a base end.

The removable cap, the needle hub, and the shield 108 may be made from conventional materials.

The shield can include a plurality of sidewalls.

The shield can comprise a first, second and third sidewalls defining an interior space configured to surround the needle on at least three sides. The third sidewall can be a center wall, central wall, or rear wall positioned between the first and second sidewalls.

The first and second sidewalls can be generally parallel to one another at a base end of the shield, then taper toward one another, and then continue to be parallel to one another.

The first and second sidewalls can extend generally parallel to one another along their entire lengths or slightly taper along their entire lengths or along only part of the lengths.

The third sidewall can be contoured from a sloping base portion to a bulging portion.

The third sidewall can be generally flat or can be undulating.

An outside surface of the bulging portion can include transverse ridges to facilitate gripping the shield, such as to be pushed by a finger to close the shield over the needle.

All three sidewalls can be joined at approximately 90° angles.

The first and second sidewalls can diverge or converge along a direction away from the third sidewall and therefore not formed at 90° angles. Near the base of the shield, the first and second sidewalls can be slightly spaced from the third sidewall, forming first and second slits. The slits can enable the first and second sidewalls to be bent away, such as to deflect, from one another or relative to one another so that the spacing between the two sidewalls at the base can vary due to the deflection, such as be closer or further away from one another compared to when they are not deflected, to facilitate mounting the shield on the hub.

The end of the shield near the base end can be wider than the end of the shield near the end wall, formed by a reducer or enlarger somewhere in between the two ends.

The width of the shield can be about the same at both ends.

The sidewalls can extend approximately half the length of the shield and adjoin a shoulder portion that extends transversely inward from the edges of all three sidewalls.

A shroud can extend from the shoulder portion away from the sidewalls.

The shroud can have a substantially U-shaped cross-section.

The shroud can be closed at its end opposite the base end of the shield.

The shield can be open along one side to accommodate passage of the needle as the shield pivots with respect to the hub.

The shield can have three sidewalls, which extend the entire length of the shield with the central wall having the shoulder. The shroud can be subsumed within the three sidewalls. The end wall at the end opposite the base can be connected to all three sidewalls and formed a closed top end.

The base end of the shield can include first and second ball hinge components or balls and first and second tabs.

The balls can extend inwardly from inner surfaces of the first and second sidewalls.

The balls and the tabs can be located on the first and second sidewalls at locations further away from the end wall than the end edge of the central wall.

The tabs can extend inwardly from the inner surfaces of the first and second sidewalls.

The tabs can extend from corners of the two sidewalls at the base end opposite those from which the balls extend.

The tabs can be formed on the inner surfaces of the first and second sidewalls at locations other than the corners, such as inwardly or recessed from the corners to engage different points on the needle hub.

The balls can be spherical and can each include a projection or stub connecting the ball to the respective sidewall.

The length of each projection, if more than one ball are included, can be adjusted to control the depth that the ball attached thereto projects into a corresponding socket, as further discussed below.

The balls can be elongated, similar to tear drops or water drops.

Each ball may include surface features, such as ridges, fins, dimples, or projections, which can alter surface contacts between the balls and the sockets to which they connect.

The balls can be configured to be received within sockets located on the hinge component of the needle hub to form a ball-and-socket hinge for pivotal movement of the shield and hub with respect to one another. The opening of the socket can be smaller than the largest diameter of the ball so as to increase the force required to separate the ball from the socket after assembly.

A first hinge component can be formed with the shield for attaching to a second hinge component formed with the needle hub.

The two sockets can share a common bore.

The two sockets can be isolated from one another, such as by a wall, a gap, or a channel such that the hinge component can be thought of as comprising two separate components for receiving the two balls.

Two cups or sockets may be provided to receive two balls. The two cups can each include a round bore and a curved end wall to close the bore. The two curved end walls of the two cups can be spaced from one another.

The hinge can be understood to include two or more separately formed components joined together to form a movable hinge. The hinge can include a first hinge component attached to a second hinge component.

The hinge can comprise a ball-and-socket joint.

Two ball-and-socket joints can be provided to form the hinge. The hinge can be characterized as a ball-and-socket hinge comprising two ball-and-socket joints, which include two balls and two sockets.

The needle hub can include a first hinge part or component and the shield can include a second hinge part or component that engages the first hinge part to pivotably secure the shield to the needle hub. The first hinge part can comprise a hinge component having sockets, which may have a common bore or be spaced from one another by a gap or a wall. The second hinge part can comprise balls, such as two spaced apart balls.

The shield can be assembled to the hub by flexing the first and second sidewalls away from each other in the area of the base end of the shield.

The slits between the first and second sidewalls and the third sidewall can facilitate the flexing.

The shield and the needle hub can be positioned such that the balls are just outside the openings of the two sockets.

The sidewalls can be forced inward so that the balls squeeze through the openings and into the sockets.

The perimeters defining two openings can be smaller than the maximum outside diameters of the two balls so that the balls and/or the perimeters defining the openings deform during the engagement.

The two perimeters can have the same opening diameter and the two balls can have the same outside diameters.

The diameters of the perimeters can be different from one another and the diameters of the two balls can be different from one another.

One or more openings may be provided with the base at the sockets for relief but can be omitted.

The shield can be positioned at an appropriate angle relative to the hub so that the tabs and/or sidewalls do not interfere with the hub during the assembly.

The shield and hub may be positioned such that their longitudinal axes are perpendicular to one another during assembly.

To use the hinged shield assembly, an operator may begin by first removing the overwrap.

The operator may then pivot the shield away from the cap and removes the cap to expose the needle. The ball-and-socket hinge may enable the shield to be retained at any desired angle with respect to the needle without the need for the operator to hold the shield on one hand while using the assembly with the other hand.

Because of the interference fit between the balls and the sockets, the tight fitting arrangement allows the interior surfaces of the sockets to grip the balls at any number of angles of the shield relative to the needle hub. Thus in use, the operator's fingers can be made available to perform other tasks without having to hold the shield in the open position or in any particular position while attempting to use the assembly. The relative sizes and/or shapes of the balls and the sockets can be tailored to provide desired relative motion and interference, or even non-interference, which is less preferred. For example, these components can be manufactured for an interference fit so that friction and interference between the moving parts retains the shield at the desired angle after the operator releases the shield.

Each ball may have a diameter that is approximately equal to the maximum diameter of each socket, but greater than the diameter of the opening. The balls and/or the perimeters defining the openings of the sockets can deform in order for the assembly to occur.

The balls can be sized to be larger than the sockets to form an interference fit. The balls can sit within the sockets and resist withdrawal from the sockets because the opening has a smaller diameter than the largest diameter of each ball.

The openings to the sockets can have a diameter that is smaller than the internal diameters of the sockets. At the opening, the opening diameter can have a first dimension, and inside the opening at the socket, the bore diameter of the socket can have a second dimension larger than the first dimension at the opening.

The relative sizes of the balls and sockets can be tailored to provide a desired amount of resistance to rotation of the balls within the sockets and degree of interference. The shapes of the balls can be tailored to provide a desired smoothness of motion, or lack of smoothness.

Alternative ball shapes can include various regular and irregular polyhedrons having a plurality of faces. Regular polyhedrons can include dodecahedron, icosahedron, octahedron, or any other polyhedron. Each of these shapes includes a plurality of faces having congruent shapes. In an irregular polyhedron, the faces may not have congruent shapes, and the faces may extend partially or fully around the circumference of the ball.

The alternative shapes can provide a plurality of faces that form planes extending perpendicularly to the first and second sidewalls. The faces may extend partially or fully around the circumference of the ball. Corresponding faces may be provided in the socket so that as the shield pivots the faces on the ball sequentially abut the faces in the socket. The resulting motion provides a ratchet-like effect, which can be thought of as tactile feedback, which enables the shield to be held at a variety of different angles with respect to the needle and provide feedback as the shield rotates.

The balls can have dimples. The dimples can form discrete contacts with the surfaces of the sockets when rotating the shield relative to the needle hub.

The shield may further comprise at least one hook that extends inwardly from the third sidewall or rear wall and/or from the shroud, such as from the center wall of the shroud.

When the entire shield is simply identified as having three sidewalls extending the entire length of the shield, the at least one hook can extend from the third sidewall from any number of points along the length of the third sidewall towards the open space defined by the gap between the first and second sidewalls.

One or more hooks can extend from one of the two sidewalls rather than the rear wall.

The two hooks can be formed integrally with the shield, such as being singularly molded with the third sidewall.

The hooks can be configured to capture the needle after use when the shield is pivoted toward the needle to secure the shield in the needle-protected position or simply protected position.

Tabs may be used to removably secure the shield to the needle hub to provide stability.

The shield may also be formed without hooks. The hooks may be omitted and the securement may be between the shield and the needle hub. A permanent secure means can prevent separation between the shield and the needle hub after their engagement and following use of the needle.

The hooks can automatically catch the needle upon closing the shield over the needle. As the shield pivots toward the needle, a ramped or rounded end surface of each hook can contact the needle, flexing the hook laterally to allow the hook to snap around and capture the needle.

The location of the hooks may be changed so that the hooks secure different sections of the needle and/or the needle hub.

One hook can snap and lock against the central post holding the needle while a different hook can lock against the needle.

One hook may be used to secure against the central post while tabs are used to secure against the transverse wall base on the needle hub.

Tabs are formed on the interior of the sidewalls and configured to be received beneath ledges comprising side edges on the needle hub to secure the shield to the needle hub in the needle-protected position.

On the corners opposite the sockets, the hub can include side edges having notches on the side of the transverse base opposite the sockets.

The transverse base and the notches can be formed as recesses on the planar surface.

The notches can be sized and shaped to be complementary to the tabs on the shield.

The tabs can snap into the notches when the shield is pivoted toward the protected position to hold the shield in that position. Attempts to open the shield can be resisted by the tabs abutting against the notches. The engagement can be separable by prying the tabs from the notches.

When closing the shield over the needle, the two sidewalls can deflect and the deflection can be aided by the slits formed on the shield when the tabs snap into the notches.

The surfaces of the ledges on the transverse base on the side opposite the notches can be ramped or tapered so that as the shield pivots toward the protected position, the tabs 136 can slide over the ramp surfaces and deflect outwardly, such as by camming action, and the two sidewalls expand outwardly as they slide thereover.

When the tabs slide around the ledges, the two sidewalls can recoil to snap the two tabs into the notches to secure the shield to the needle hub in a protected position. This securement by the tabs against the notches can stabilize the shield when an attempt is made to push the shield back open, which is stronger and more stable compared to the shield only hooking onto the needle.

The locking action provided by the tabs, the notches, and the ledges can be reversible.

The first and second sidewalls can flex away from one another. By flexing the sidewalls sufficiently, the tabs can clear the notches and the ledges so that the shield can be pivoted away from the protected position.

The hook or hooks extending from the third sidewall can form locking mechanisms that are more difficult if not impossible to reverse when they hook around the needle and/or the central post on the needle hub. Due to the confined space inherent in the design, these hooks may provide little access to enable un-hooking once they engage and therefore serve to lock the shield to the needle hub.

The needle hub can include an elongated portion and a transverse base portion.

The transverse base portion can substantially bisect the elongated portion, defining a proximal elongated portion and a distal elongated portion.

Both the proximal and distal elongated portions can be generally cylindrical. The proximal cylindrical portion can be shaped as a smooth hollow cylinder with a female Luer and includes external threads at its proximal end.

The threads can be configured to engage a female thread at a distal end of a syringe to secure the hinged cap device to a male tip.

The male tip can be a Luer tip of a syringe.

The distal cylindrical portion can include a hollow cylinder defining the central post for holding a needle.

The hollow cylinder can comprise one or more fins extending outwardly and parallel to an axis of the cylinder. The one or more fins can be used as retention features for the removable cap to engage, such as to frictionally grip.

The hollow cylinder can be configured to receive the blunt proximal end of a needle in a glued engagement with the needle disposed inside the cylinder.

The engagement with the needle and the hollow cylinder can be a press-fit engagement.

The transverse base portion can include a substantially planar portion or flange bisecting the two cylindrical portions, at least along external surfaces, and has, at one end or one edge, a hinge component, which can be a substantially cylindrical portion.

The cylindrical portion on the flange may optionally incorporate a projection or a physical barrier on an exterior thereof to serve as a shield stop to limit how far the shield can rotate away from the needle in an open position.

The projection on the cylindrical portion may act as a spacer to restrict the amount of rotation of the shield from the position over the needle to a position rotated away from the needle.

The cylindrical portion can comprise first and second socket parts or joints of the ball-and-socket hinge.

Each socket part may be referred to as a cup-like depression, such as a socket, for receiving a correspondingly shaped ball.

Each socket part may include a perimeter defining an opening having a beveled rim.

The diameter of the socket part can vary from a relatively narrow opening, with the diameter increasing inward of the opening and then gently tapering down to a more narrow diameter.

The variable diameter opening can be configured to receive a ball of the ball and socket hinge.

The socket can have a variable interior surface contour to create varying interference with the ball when the ball is received therein.

A socket that forms fit with a ball in a ball-and-socket joint can be useable as a hinge in a hinge cap device of the present disclosure and wherein an interference between the hinge components can also be applicable in a ball and socket joint.

The interference fit can be appropriate to enable the shield to be self-supporting at any number of open position or angular position and remain in that position when rotated without freely swinging or rotating due to gravity and without requiring external support.

A ball-and-socket joint can be rotated along multiple axes and have built-in support bearings.

The substantially planar portion or flange can comprise one or more stop pieces.

A single stop piece can be incorporated to stop the shield from over rotation when closing the shield over the needle following use.

Two or more stop pieces may be incorporated on opposite sides of the transverse base.

The flange can have a side edge comprising a stop piece.

The flange can have two side edges, each with a stop piece for a total of two.

The two stop pieces can be generally in the form of a parallelepiped base or simply base.

The two stop pieces can extend radially outward relative to a lengthwise axis of the needle hub, from the substantially planar portion of the two side edges, on a side or sides of the assembly adjacent to the hinge component.

The stop pieces can be placed in the rotational path of the first and second sidewalls of the shield as the shield rotates around the hinge component.

The parallelepiped base of the stop pieces can be formed with squared off edges, tapered or rounded corners.

The top of the parallelepiped base can be even with the top of the substantially planar portion or flange, but not as deep or thick as the substantially planar portion.

The stop pieces may not be as wide as the substantially planar portion or flange.

Each stop piece may occupy a section of the side edge of the transverse base and be less than or equal to a width of the transverse base.

The top surface of each stop piece can be offset from the top of the planar portion.

The stop pieces can extend radially outwardly a greater distance than the width of the shield measured between the two exterior surfaces of the sidewalls. The stop pieces can stop the shield from over-rotation even if the shield spreads or widens, such as distort, upon contacting the stop pieces.

The stop pieces can extend radially outwardly a shorter of the distance between the two sidewalls.

Once the shield contacts the at least one stop piece, the base end of each of the sidewalls can be prevented from moving past, such as further rotating, beyond an at rest point when the shield is in a protected position in one rotational direction.

In the protected position, the shield can cover the needle and locks against either the needle, the central post or distal cylindrical portion, or the needle hub, such as the transverse base.

The substantially planar portion can incorporate two stop pieces, each of which comprises a planar surface to abut the shield.

The stop pieces can each includes a flat distal side, which is configured to make contact a proximal edge or contact edge of the shield.

The stop piece can include rounded edges on the proximal side of the stop piece.

The stop pieces may be placed or positioned along the two side edges of the planar portion or flange, such as anywhere between the notch and the socket.

The locations of the distal surfaces of the two stop pieces relative to the flange may vary depending on where the contact edges of the first and second sidewalls contact the stop pieces during closing rotation of the shield.

The placement of the stop pieces may be selected in conjunction with the design and placement of the hooks, the tabs, and the notches so that collectively they allow locking of the shield to the needle hub without interference.

The stop pieces and the first and second sidewalls of the shield can have latching or locking detents so that when the sidewalls abut the stop pieces, the latching or locking detents prevent the shield from separating from the hub and re-open to expose the needle.

The needle assembly can have a needle hub and a shield connected to the needle hub in a ball-and-socket arrangement.

The transverse base portion can include two stop pieces, on opposite sides of the substantially planar side edges and corresponding to the sides of the two sidewalls.

The stop pieces can each includes a parallelepiped base, or simply a base, similar to the stop piece.

Each base can be viewed as a radially extending member in that it extends radially of the lengthwise axis of the needle.

The stop pieces can each include a longitudinally extending member, capture element, or capture portion.

The capture elements or portions can extend directly from the two respective bases.

The base and the capture portion of each stop piece can form a generally "L" shape or "T" shape structure when viewing from an end.

Each capture portion can be viewed as an axially extending member in that it extends along the same axial direction as the lengthwise axis of the needle.

The capture portions on the two stop pieces can be sized and shaped to overlap a reduced depth portion of the shield sidewall. The reduced depth portion of each sidewall can extend transversely, beginning near the hinge component and ending near the middle of the each of the first and second sidewalls and can have a dimension that is smaller or less than other depth portions, such as being thinner in dimension compared to other wall surfaces of the same sidewall.

The base and the capture portion of each stop piece can define a gap or retention space for accommodating the reduced depth portion of the respective sidewall.

The two capture portions of the two stop pieces can restrict the first and second sidewalls from deflecting radially outwardly away from the lengthwise axis of the needle hub to slip past the two stop pieces and over-rotate.

A single stop piece having a base and a capture portion can be incorporated with the transverse base portion.

The present stop pieces can not only stop the shield from over-rotation by providing radially extending stop surfaces to interfere with the two sidewalls as they rotate, but can also prevent or limit outward radial deflection of the two sidewalls by capturing the two sidewalls within retention spaces defined at least in part by the two capture portions.

Each stop piece can comprise a radially extending member and an axially extending member.

The base can form a radially extending member and the capture portion can form an axially extending member, both relative to a lengthwise axis of the needle hub or the needle.

At least one of the plurality of sidewalls of the shield can contact the at least one stop piece to prevent over rotation of the shield in one rotational direction when the shield is in a protected position over the needle.

Means for limiting outward radial deflection of at least one of the plurality of sidewalls of the shield, can include a capture portion extending from a base on a stop piece 190, the bases of the stop pieces extending sufficiently radially so that the corresponding sidewalls on the shield can be received within the retention spaces without the need for incorporating reduced depth portions on the sidewalls, and the edge of each sidewall can be enlarged and the retention spaces can be sized and shaped to accommodate the enlarged sections of the sidewalls.

As he tabs on the shield can come into contact with the substantially planar portion of the hub, the width of the tabs relative to the width of the planar portion can cause the sidewalls on the shield to deflect transversely outwardly, away from the lengthwise axis of the needle and radially of the lengthwise axis of the needle hub. The radial deflection of the two sidewalls can be greatest at the tabs and decrease as it moves towards the end of the first and second sidewalls with the third sidewall.

The reduced depth portion of each of the first and second sidewalls can slide pass the distal most end of the capture portion, between an inside edge of the capture portion and the corresponding side edge of the planar portion transversely opposite the inside edge.

The process of passing the reduced depth portion between the distal most end of the capture portion between the inside edge of the capture portion and a side of the substantially planar portion can capture the respective sidewall in the retention space. The capture portion can capture the reduced depth portion within the retention space as the shield rotates to close over the needle.

The radially extending portion of each stop piece can be sufficiently long or can sized and shaped so that a gap or clearance can be provided to ensure sufficient space for the reduced depth portion of the respective sidewall to slide within the retention space. There can be sufficient clearance or tolerance within the retention space to accommodate the respective sidewall of the shield as the sidewall slides inside the retention space.

The contact edge of each sidewall adjacent the reduced depth portion and the distal face of the capture portion of the stop piece can be configured to contact the shield in the shield 108 lock position by providing an increased depth section. The increased depth section can increase the thickness of each respective sidewall, making the sidewall thicker at and near the base section of the sidewall.

The increased depth section can start at a transverse line distal of the proximal edge of the sidewall and transition from this point away from the sidewall, increasing the thickness such that a rounded proximal edge extends outside of the stop piece.

At an end point of the contact edge closest to the hinge component, the contact edge can be generally parallel to the proximal surface of the substantially planar portion.

At a halfway point along the contact edge, the contact edge surface can be generally parallel to the proximal surface of the substantially planar portion.

Just past the halfway point in the direction away from the hinge component 164, the contact edge can begin to curve, and follow a geometrically proximally curving path. As the path of the contact edge moves away from the hinge component and towards the tab, the contact edge can curve increasingly proximally.

The distal face of the capture portion of the stop piece can be curved to match the curve of the contact edge along the distal face of the capture portion.

The contact edge can remain generally parallel to the proximal surface of the substantially planar portion for its entire length, with an angled transition at the opposite end. The distal face of the capture portion can be sized and shaped to match the contact edge.

The entire lower portions of the first and second sidewalls can be sufficiently narrow or thin to ensure passing into the retention spaces without the contoured surfaces or with less contoured surfaces described.

The shield can pivot to cover the needle when a force is applied to the shield, such as by using digital pressure or pushing the shield against a surface to rotate the shield to a closed position.

The shield can pivot until the hooks snap around the needle and/or the tabs snap into the notches on the needle hub.

The shield 108 can be secured to the needle hub by engaging the tabs on the shield with the notches on the needle hub.

A hook may be provided to hook onto the needle and/or to hook onto the central post of the needle hub.

The stop pieces and the first and second sidewalls can also incorporate locking detents, as previously discussed, to secure the shield to the needle hub.

When the shield reaches the closed position or protected position, the shield can be prevented from rotating in the opposite direction, or open direction to re-expose the needle, by the engagement of the tabs with the notches and/or by the hook hooking onto the needle or the post.

In the same closed position or in the process of closing the shield over the needle, the shield can be prevented from over rotating by the contact between the stop pieces and the contact edges of the shield.

One or more stop pieces can be used to prevent the shield from over-rotating.

### BRIEF DESCRIPTION OF THE DRAWINGS

The various embodiments of the present hinged shield assemblies now will be discussed in detail with an emphasis on highlighting the advantageous features. These embodiments depict the novel and non-obvious hinged shield assemblies shown in the accompanying drawings, which are for illustrative purposes only. These drawings include the following figures, in which like numerals indicate like parts:
Figure 1 is a side cross sectional view of a hinged needle assembly in a packaged position with a removable cap;
Figure 2 is a plan view of a shield;
Figure 3 is a partial side perspective view of another embodiment of the present hinged needle assemblies;
Figure 4 is a top view of the hinged shield assembly of Figure 3;
Figure 5 is a perspective view of an embodiment of a hub in accordance with the present hinged needle assemblies;
Figure 6 is a perspective view of another embodiment of a hub of the present hinged needle assemblies;
Figure 7 is a perspective view of yet another embodiment of a hub of the present hinged needle assemblies;
Figure 8 is a perspective view of another embodiment of the present hinged needle assemblies; and
Figure 9 is a perspective view of yet another embodiment of the present hinged needle assemblies.

### DETAILED DESCRIPTION

The following detailed description describes the present embodiments, including apparatuses, devices, and methods, with reference to the drawings. In the drawings, reference numbers label elements of the present embodiments. These reference numbers are reproduced below in connection with the discussion of the corresponding drawing features.

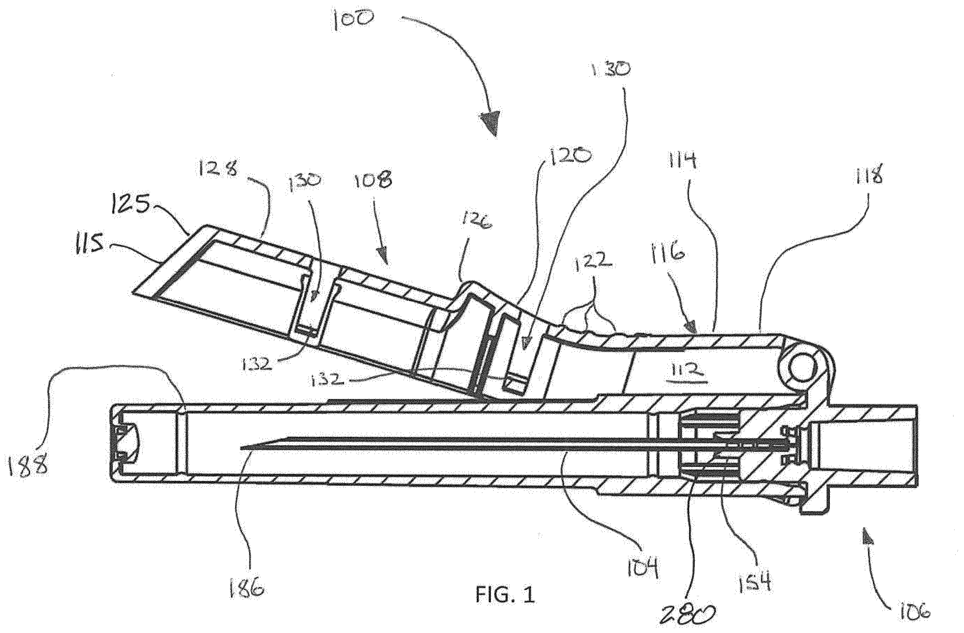

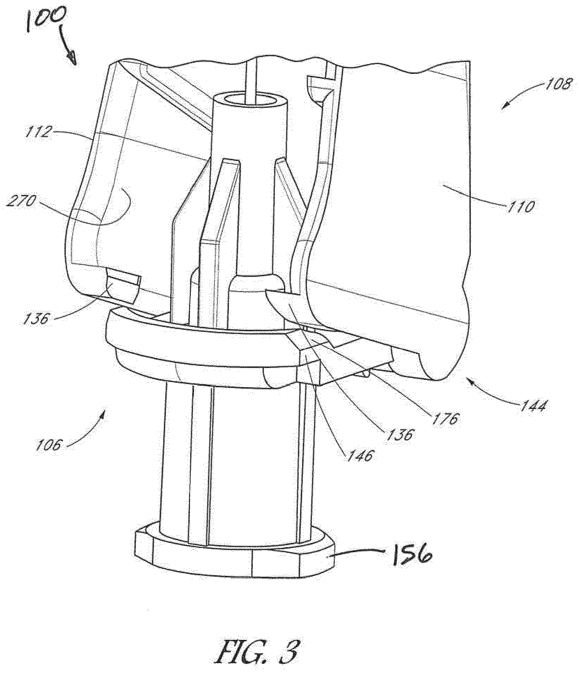

Figures ("FIGs.") 1-9 illustrate different variations or embodiments of hinged shield assemblies and their components in accordance with aspects of the invention. The hinged shield assemblies described herein may alternatively be referred to as hinged needle assemblies, hinged cap assemblies, or hinged cap devices. As shown in Figure 1, the hinged shield assembly 100 comprises a shield 108 for shielding a needle 104 extending from a needle hub 106. In the example shown, the needle 104 is secured to a central post or cylinder post 280 formed with the needle hub 106. In another example, the needle hub 106 does not include a needle but comprises a male Luer where the central post 280 is located for receiving a separately formed female Luer hub having a needle attached to the separately formed needle hub. The needle hub 106 shown in FIG. 1 has a female Luer with external threads 156 (FIG. 3) for receiving a male Luer, optionally with a threaded collar. With reference to Figures 1 and 3, the shield 108 is pivotable about the hub 106 to expose the needle 104 for performing a medical procedure and to cover the needle 104 following use. As described in further detail below, the shield 108 is configured to be manipulated from a packaged position (Figure 1, similar to when the device is packaged in a plastic overwrap) to a ready-to-use position in which the needle is exposed, from the ready-to-use position to an open position in which the shield is rotated clear of the needle for use, and from the open position to a secured position or protected position in which the shield 108 is rotated to cover the needle 104 and locks to the needle and/or to the needle hub 106 to prevent or deter re-exposing the needle after it has been used.

Figure 1 shows the assembly 100 in a packaged position, which typically includes a conventional blister pack for packaging the assembly therein. When the assembly 100 is manufactured and packaged, it includes a removable cap 188 for capping the sharp distal tip or needle tip 186 (Figure 1) of the needle 104 prior to use and for packaging. The tapered removable cylindrical cap 188 fits over the distal cylindrical portion 154 of the needle hub 106 in a friction fit. The cap 188 is sufficiently long to accommodate the length of the needle 104 and can be long enough so as to fit over several different sized, such as different lengths, needles. An outer diameter of the cap 188 is sized so that the width of the shield 108 can only extend around part of the removable cap 188, enabling the base end 116 of the rear wall 114 of the shield 108 to be spaced from the cap 188 and the shield 108 capable of pivoting towards the cap 188 as shown in Figure 1, in which the shield 108 partly covers the cap 188. Each sidewall has a base end 116. In this configuration, the assembly 100 occupies a relatively smaller profile, such as a relatively smaller space, for shipping as compared to a configuration in which the shield 108 extends perpendicularly to the cap 188 or not overlap with any part of the removable cap 188. The removable cap 188, the needle hub 106, and the shield 108 may be made from conventional materials.

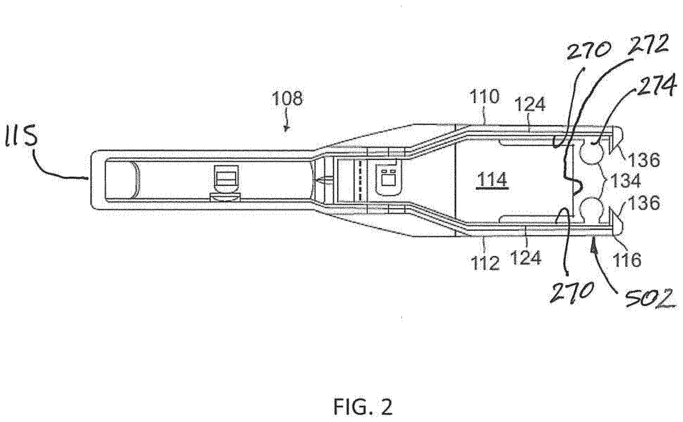

With reference to Figures 2, 8 and 9 in addition to Figure 1, the shield 108 comprises a plurality of sidewalls, including first, second and third sidewalls 110, 112, 114 defining an interior space configured to surround the needle 104 on at least three sides. The third sidewall 114 may also be referred to as a center wall, central wall, or rear wall and is positioned between the first and second sidewalls 110, 112. With reference to Figures 1, 4, 8 and 9, the first and second sidewalls 110, 112 are generally parallel to one another at a base end 116 of the shield 108, then taper toward one another, and then continue parallel to one another. Other sidewall contours are contemplated, such as having two sidewalls 110, 112 that extend generally parallel to one another along their entire lengths or slightly taper along their entire lengths or along only part of the lengths. With reference to Figure 1, the third sidewall 114 is contoured from a sloping base portion 118 to a bulging portion 120. The third sidewall 114 may also be generally flat or can be undulating. An outside surface of the bulging portion 120 can include transverse ridges 122 to facilitate gripping the shield 108, such as to be pushed by a finger to close the shield 108 over the needle 104. As shown in Figure 2, all three sidewalls 110, 112, 114 are joined at approximately 90° angles. In some examples, the first and second sidewalls 110, 112 can diverge or converge along the direction away from the third sidewall 114 and therefore not formed at 90° angles. Near the base 116 of the shield 108, the first and second sidewalls 110, 112 are slightly spaced from the third sidewall 114, forming first and second slits 124. The slits 124 enable the first and second sidewalls 110, 112 to be bent away, such as to deflect, from one another or relative to one another so that the spacing between the two sidewalls 110, 112 at the base can vary due to the deflection, such as be closer or further away from one another compared to when they are not deflected, to facilitate mounting the shield 108 on the hub 106 (Figure 1) during manufacture, as further described below. As shown in FIG. 3, the end of the shield 108 near the base end 116 is wider than the end of the shield 108 near the end wall 115, formed by a reducer or enlarger somewhere in between the two ends 115, 116. In other examples, the width of the shield 108 is about the same at both ends 115, 116.

With reference again to Figure 1, the sidewalls 110, 112, 114 extend approximately half the length of the shield 108 and adjoin a shoulder portion 126 that extends transversely inward from the edges of all three sidewalls 110, 112, 114. A shroud 128 extends from the shoulder portion 126 away from the sidewalls 110, 112, 114. The shroud 128 has a substantially U-shaped cross-section. The shroud 128 is closed at its end opposite the base end 116 of the shield 108. The shield 108 is open along one side to accommodate passage of the needle 104 as the shield 108 pivots with respect to the hub 106, as explained in detail below. The shield 108 may also be viewed as having three sidewalls 110, 112, 114, that extend the entire length of the shield with the central wall 114 having the shoulder 126. The so-called shroud 128 is subsumed within the three sidewalls 110, 112, 114. The end wall 115 at the end opposite the base 116 is connected to all three sidewalls and formed a closed top end 125.

With reference again to Figure 2, the base end 116 of the shield 108 includes first and second ball hinge components or balls 134 and first and second tabs 136. The balls 134 extend inwardly from inner surfaces 270 of the first and second sidewalls 110, 112. In an example, the balls 134 and the tabs 136 are located on the first and second sidewalls 110, 112 at locations further away from the end wall 115 than the end edge 272 of the central wall 114. The tabs 136 can also extend inwardly from the inner surfaces 270 of the first and second sidewalls 110, 112. In an example, the tabs 136 extend from corners of the two sidewalls 110, 112 at the base end 116 opposite those from which the balls 134 extend. In some examples, the tabs 136 can be formed on the inner surfaces 270 of the first and second sidewalls at locations other than the corners, such as inwardly or recessed from the corners to engage different points on the needle hub 106. In an example, the balls 134 are spherical and each includes a projection or stub 274 connecting the ball to the respective sidewall. The length of each projection 274, if more than one ball 134 are included, can be adjusted to control the depth that the ball attached thereto projects into a corresponding socket, as further discussed below. In other examples, the balls 134 are elongated, similar to tear drops or water drops. Each ball may optionally include surface features, such as ridges, fins, dimples, or projections, which can alter surface contacts between the balls and the sockets to which they connect, as further discussed below.

With reference to Figures 3, 4 and 5, the balls 134 are configured to be received within sockets 142 located on the hinge component 164 of the needle hub 106 to form a ball-and-socket hinge 144 for pivotal movement of the shield 108 and hub 106 with respect to one another. Thus, the present disclosure is understood to include a first hinge component formed with the shield 108 for attaching to a second hinge component formed with the needle hub 106. In one example, the two sockets 142 share a common bore. In another example, the two sockets 142 are isolated from one another, such as by a wall, a gap, or a channel such that the hinge component 164 can be thought of as comprising two separate components for receiving the two balls 134. For instance, two cups or sockets 142 may be provided to receive two balls 134. The two cups can each include a round bore and a curved end wall to close the bore. The two curved end walls of the two cups can be spaced from one another. Thus, the hinge 144 can be understood to include two or more separately formed components, i.e., a multi-piece structure, that are joined together to form a movable hinge. The hinge 144 is understood to include a first hinge component attached to a second hinge component. The hinge can comprise a ball-and-socket joint 166. As shown, two ball-and-socket joints 166 are provided to form the hinge 144 of the present disclosure. The hinge 144 can be characterized as a ball-and-socket hinge comprising two ball-and-socket joints 166, which include two balls 134 and two sockets 142. More generally speaking, the needle hub is understood to include a first hinge part or component 500 and the shield includes a second hinge part or component 502 that engages the first hinge part to pivotably secure the shield 108 to the needle hub 106. The first hinge part can be understood to comprise a hinge component having sockets, which may have a common bore or be spaced from one another by a gap or a wall. The second hinge part can be understood to comprise balls, such as two spaced apart balls.

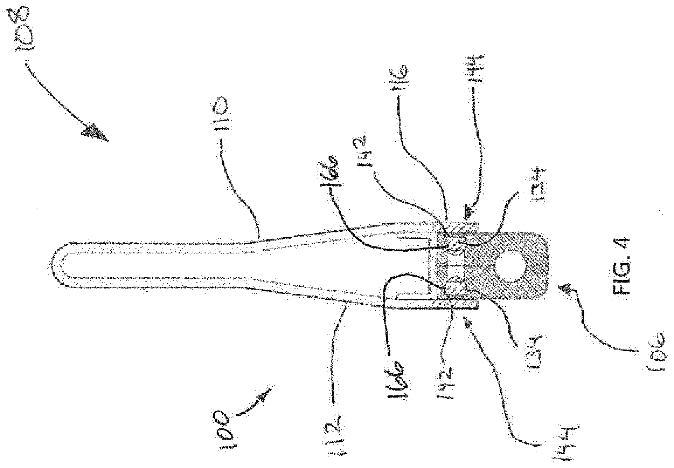

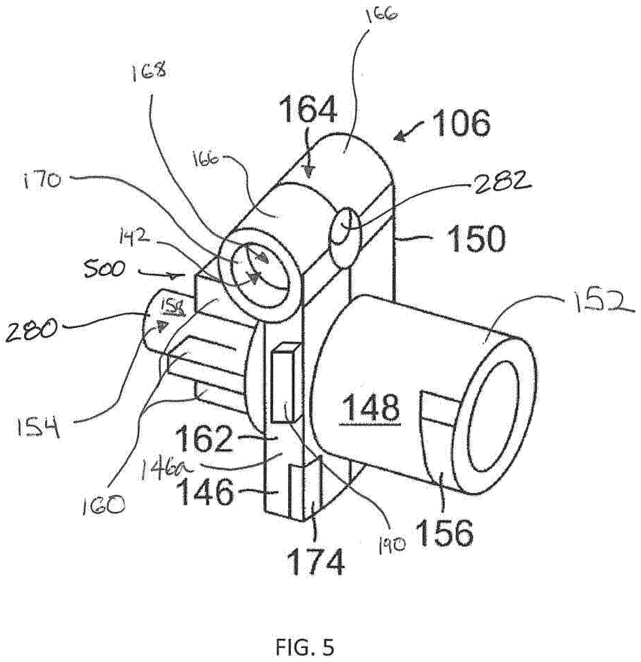

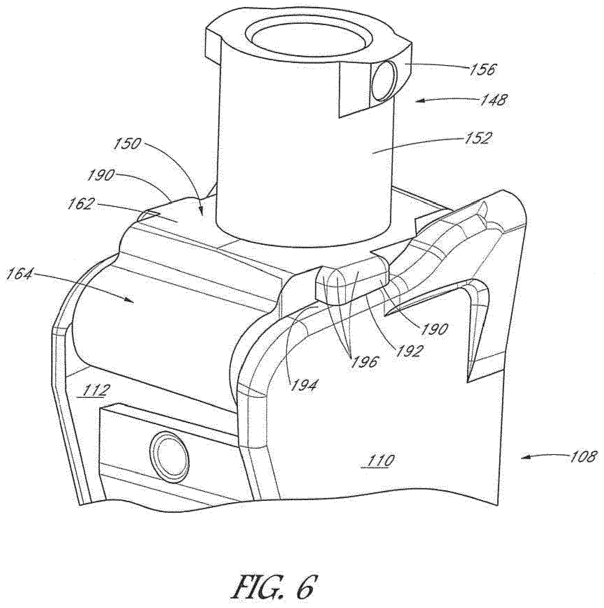

When the assembly 100 is manufactured, the shield 108 is assembled to the hub 106 by flexing the first and second sidewalls 110, 112 (Figures 2 and 3) away from each other in the area of the base end 116 of the shield 108. The slits 124 (Figure 2) between the first and second sidewalls 110, 112 and the third sidewall 114 facilitate the flexing. The shield 108 and the needle hub 106 are then positioned such that the balls 134 are just outside the openings 168 (Figure 5) of the two sockets 142. The sidewalls 110, 112 are then forced inward so that the balls 134 squeeze through the openings 168 and into the sockets 142. The perimeters defining two openings 168 are smaller than the maximum outside diameters of the two balls so that the balls and/or the perimeters defining the openings deform during the engagement. In one example, the two perimeters have the same opening diameter and the two balls have the same outside diameters. In other examples, the diameters of the perimeters are different from one another and the diameters of the two balls are different from one another. One or more openings 282 may be provided with the base at the sockets 142 for relief but can be omitted, as shown in FIG. 6. As shown in Figure 3, as these steps are performed, the shield 108 is positioned at an appropriate angle relative to the hub 106 so that the tabs 136 and/or sidewalls 110, 112 do not interfere with the hub 106 during the assembly. For example, the shield 108 and hub 106 may be positioned such that their longitudinal axes are perpendicular to one another, as shown in Figure 4, during assembly.

To use the present hinged shield assembly 100, an operator typically begins with the assembly 100 in the packaged configuration of Figure 1, such as by first removing the overwrap. The operator then pivots the shield 108 away from the cap 188 and removes the cap 188 to expose the needle 104. The assembly 100 is then ready to be used to inject medication or to draw blood. Advantageously, the ball-and-socket hinge 144 enables the shield 108 to be retained at any desired angle with respect to the needle 104 without the need for the operator to hold the shield 108 on one hand while using the assembly with the other hand. For example, because of the interference fit between the balls 134 and the sockets 142, the tight fitting arrangement allows the interior surfaces of the sockets 142 to grip the balls 134 at any number of angles of the shield relative to the needle hub 106. Thus in use, the operator's fingers are available to perform other tasks without having to hold the shield 108 in the open position or in any particular position while attempting to use the assembly 100. The relative sizes and/or shapes of the balls 134 and the sockets 142 can be tailored to provide desired relative motion and interference, or even non-interference, which is less preferred. For example, these components can be manufactured for an interference fit so that friction and interference between the moving parts retains the shield 108 at the desired angle after the operator releases the shield 108.

The diameter of the sockets 142 that receive the balls 134, as shown in Figure 4, may be changed in a number of ways. Each ball 134 has a diameter that is approximately equal to the maximum diameter of each socket 142, but greater than the diameter of the opening 168 (Figure 5). Thus, in assembling the balls to the sockets, the balls and/or the perimeters defining the openings of the sockets deform in order for the assembly to occur. The balls can be sized to also be larger than the sockets to form an interference fit. The balls 134 thus sit within the sockets 142 and resist withdrawal from the sockets 142 because the opening 168 has a smaller diameter than the largest diameter of each ball 134. The openings to the sockets have a diameter that is smaller than the internal diameters of the sockets. In other words, right at the opening 168, the opening diameter has a first dimension and inside the opening 168 at the socket, the bore diameter of the socket has a second dimension, which is larger than the first dimension at the opening. The relative sizes of the balls 134 and sockets 142 can be tailored to provide a desired amount of resistance to rotation of the balls 134 within the sockets 142 and degree of interference. Also, the shapes of the balls 134 can be tailored to provide a desired smoothness of motion, or lack of smoothness, as described below.

In alternative embodiments, alternative shapes for the outer surfaces of the balls are provided. Possible alternative shapes include various regular and irregular polyhedrons having a plurality of faces. Examples of regular polyhedrons include dodecahedron, icosahedron, octahedron, or any other polyhedron. Each of these shapes includes a plurality of faces having congruent shapes. In an irregular polyhedron the faces do not have congruent shapes, and the faces may extend partially or fully around the circumference of the ball. These alternative shapes provide a plurality of faces that form planes extending perpendicularly to the first and second sidewalls 110, 112. The faces may extend partially or fully around the circumference of the ball 134. Corresponding faces may be provided in the socket 142 so that as the shield 108 pivots the faces on the ball 134 sequentially abut the faces in the socket. The resulting motion provides a ratchet-like effect, which can be thought of as tactile feedback, which enables the shield 108 to be held at a variety of different angles with respect to the needle 104 and provide feedback as the shield rotates. In other examples, the balls 134 have dimples, similar to a golf ball.

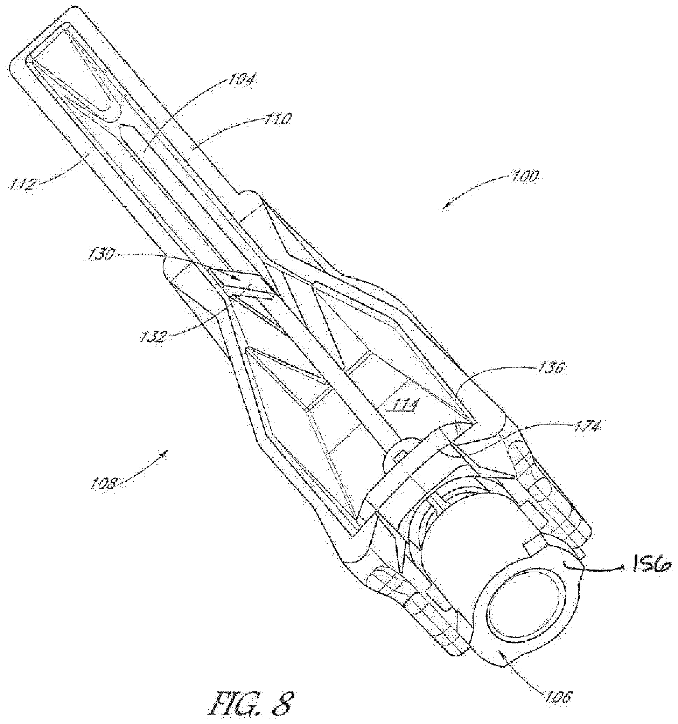

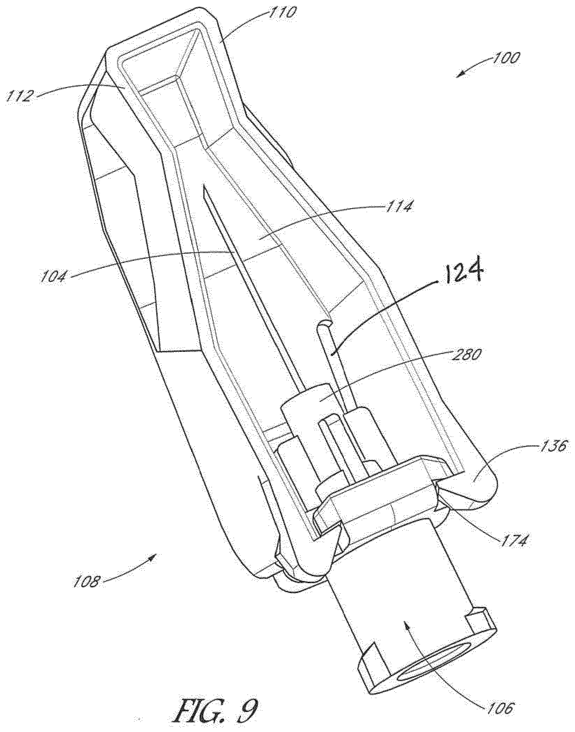

With reference to Figure 8 in addition to Figure 1, the shield 108 further comprises at least one hook 130 that extends inwardly from the third sidewall or rear wall 114 and/or from the shroud 128, such as from the center wall of the shroud. But when the entire shield 108 is simply identified as having three sidewalls extending the entire length of the shield as previously described, the at least one hook 130 extends from the third sidewall from any number of points along the length of the third sidewall towards the open space defined by the gap between the first and second sidewalls 110, 112. In some examples, one or more hooks 130 can extend from one of the two sidewalls 110, 112 rather than the rear wall. In the embodiment illustrated in Figure 1, the two hooks 130 are formed integrally with the shield 108, such as being singularly molded with the third sidewall 114. The hooks 130 are configured to capture the needle 104 after use when the shield 108 is pivoted toward the needle 104 to secure the shield 108 in the needle-protected position or simply protected position, which is discussed in more detail below. In addition to the one or more hooks, tabs 136 may be used to removably secure the shield 108 to the needle hub 106 to provide stability. The shield 108 may also be formed without hooks as shown in Figure 9. For example, when used with a small diameter needle gauge, the hooks may be omitted and the securement may be between the shield 108 and the needle hub 106. In embodiments without any hook 130, more permanent secure means may be incorporate to prevent separation between the shield 108 and the needle hub 106 after their engagement and following use of the needle.

The hooks 130 automatically catch the needle 104 upon closing the shield 108 over the needle 104. As the shield 108 pivots toward the needle 104, a ramped or rounded end surface 132 of each hook 130 contacts the needle 104, flexing the hook 130 laterally to allow the hook 130 to snap around and capture the needle 104. In other examples, one hook or more than one hook may be incorporated, such as that shown in FIG. 1 with two hooks. In still other examples, the location of the hooks may be changed so that the hooks secure different sections of the needle and/or the needle hub. For example, one hook can snap and lock against the central post 280 holding the needle while a different hook can lock against the needle. In still other examples, one hook is used to secure against the central post while tabs are used to secure against the transverse wall base on the needle hub 106.

With reference again to Figure 3, tabs 136 are formed on the interior of the sidewalls 110, 112 and configured to be received beneath ledges 146 comprising side edges on the needle hub 106 to secure the shield 108 to the needle hub 106 in the needle-protected position. These aspects are described in detail below. On the corners opposite the sockets 142 (FIG. 5), the hub 106 includes side edges 146a having notches 174 on the side of the transverse base 150 opposite the sockets 142. The transverse base 150 and the notches 174 are formed as recesses on the planar surface 162 (FIG. 5). The notches 174 are sized and shaped to be complementary to the tabs 136 on the shield 108. The tabs 136 snap into the notches 174 when the shield 108 is pivoted toward the protected position to hold the shield 108 in that position. For example, any attempt to open the shield 108 will be resisted by the tabs 136 abutting against the notches 174. However, the engagement is separable as a user can simply pride the tabs 136 from the notches 174 to separate their engagement. When closing the shield 108 over the needle 104, the two sidewalls 110, 112 deflect and the deflection is aided by the slits 124 formed on the shield 108 when the tabs 136 snap into the notches 174. With reference to Figure 3, the surfaces 176 of the ledges 146 on the transverse base 150 on the side opposite the notches 174 are ramped or tapered so that as the shield 108 pivots toward the protected position, the tabs 136 slide over the ramp surfaces 176 and deflect outwardly, such as by camming action, and the two sidewalls 110, 112 expand outwardly as they slide thereover. When the tabs 136 slide around the ledges 146, the two sidewalls 110, 112 recoil to snap the two tabs 136 into the notches 174 to secure the shield 108 to the needle hub 106 in a protected position. This securement by the tabs 136 against the notches 174 stabilizes the shield 108 when an attempt is made to push the shield 108 back open, which is stronger and more stable compared to the shield 108 only hooking onto the needle 104. However, the locking action provided by the tabs 136, the notches 174 and the ledges 146 is reversible. An operator may apply digital pressure to flex the first and second sidewalls 110, 112 away from one another. By flexing the sidewalls sufficiently, the tabs 136 can clear the notches 174 and the ledges 146 so that the shield 108 can be pivoted away from the protected position. Conversely, the hook or hooks 130 extending from the third sidewall 114 form locking mechanisms that are more difficult if not impossible to reverse when they hook around the needle 104 and/or the central post 280 on the needle hub 106. Due to the confined space inherent in the design, these hooks 130 provide little access to enable un-hooking once they engage and therefore serve to lock the shield to the needle hub.

Figures 5-7 illustrate an exemplary needle hub 106 in detail in accordance with aspects of the present invention. The exemplary needle hub 106 includes an elongated portion 148 and a transverse base portion 150. The transverse base portion 150 substantially bisects the elongated portion 148, defining a proximal elongated portion 152 and a distal elongated portion 154 (Figure 13). As shown, both the proximal and distal elongated portions are generally cylindrical. The proximal cylindrical portion 152 is shaped as a smooth hollow cylinder with a female Luer and includes external threads 156 at its proximal end. The threads 156 is configured to engage a female thread at a distal end of a syringe (not shown) to secure the hinged cap device 100 to a male tip, such as a Luer tip of a syringe. The distal cylindrical portion 154 includes a hollow cylinder 158 defining the central post 180 for holding a needle 104. The hollow cylinder 158 optionally comprises one or more fins 160 extending outwardly and parallel to an axis of the cylinder 158. When incorporated, the one or more fins 160 can also be used as retention features for the removable cap 188 (FIG. 1) to engage, such as to frictionally grip. Four evenly spaced fins 160 are shown, but any number may be provided, including less than four or greater than four. The hollow cylinder 158 is configured to receive the blunt proximal end of a needle 104 in a glued engagement with the needle 104 disposed inside the cylinder 158, as shown in Figure 1. In an alternative embodiment, the engagement with the needle can be a press-fit engagement.

The transverse base portion 150 includes a substantially planar portion or flange 162 bisecting the two cylindrical portions 148, 154, at least along external surfaces, and has, at one end or one edge, a hinge component 164, which in the present embodiment is a substantially cylindrical portion 164. The cylindrical portion 164 on the flange 162 may optionally incorporate a projection or a physical barrier on an exterior thereof to serve as a shield stop to limit how far the shield can rotate away from the needle 104 in an open position. For example, the projection on the cylindrical portion 164 may act as a spacer to restrict the amount of rotation of the shield 108 from the position over the needle to a position rotated away from the needle. In an example, the cylindrical portion 164 comprises first and second socket parts or joints 166 of the ball-and-socket hinge 144. Each socket part 166 may also be referred to as a cup-like depression, such as a socket, for receiving a correspondingly shaped ball. Each socket part 166 includes a perimeter defining an opening 168 having a beveled rim 170. From the opening 168, the diameter of the socket part 166 varies from a relatively narrow opening 168, with the diameter increasing inward of the opening 168 and then gently tapering down to a more narrow diameter. This variable diameter opening is configured to receive a ball of the ball and socket hinge. Of course, the socket 142 can have a variable interior surface contour to create varying interference with the ball when the ball is received therein. Generally speaking, a socket 142 that forms fit with a ball 134 in a ball-and-socket joint is useable as a hinge in a hinge cap device of the present disclosure and wherein an interference between the hinge components is also applicable as a ball and socket joint of the present disclosure. The interference fit is appropriate to enable the shield to be self-supporting at any number of open position or angular position and remain in that position when rotated without freely swinging or rotating due to gravity and without requiring external support. This allows a user to operate the assembly 100 without having to hold the shield 108 when obtaining a sample or when performing an injection. Ball-and-socket joints are a special class of joints that enjoy the highest freedom of motion thanks to their unique structure. For example, a ball-and-socket joint can be rotated along multiple axes and have built-in support bearings compared to a simple pin and hole arrangement, such as those typically used with a door hinge, which is limited to a single rotational axis.

As shown in Figure 5, the substantially planar portion or flange 162 can comprise one or more stop pieces 190. In an example, a single stop piece 190 is incorporated to stop the shield 108 from over rotation when closing the shield 108 over the needle 104 following use, as further discussed below. In other examples, two or more stop pieces 190 are incorporated with the hinged needle assembly 100. For example, two stop pieces 190 may be incorporated on opposite sides of the transverse base 150. In an example, the flange 162 has a side edge 146a comprising a stop piece 190. In the embodiment shown, the flange 162 has two side edges 146a, each with a stop piece 190 for a total of two with additional stop pieces contemplated. The two stop pieces 190 are generally in the form of a parallelepiped base or simply base 207 (FIG. 5), which extends outward, such as radially outward relative to a lengthwise axis of the needle hub, from the substantially planar portion of the two side edges 146a, on a side or sides of the assembly adjacent to the hinge component 164. The stop pieces 190 are placed in the rotational path of the first and second sidewalls 110, 112 of the shield 108 as the shield rotates around the hinge component 164. The parallelepiped base of the stop pieces 190 is formed with squared off edges but tapered or rounded corners are contemplated. The top of parallelepiped base can be even with the top of the substantially planar portion or flange 162, but the parallelepiped base is not as deep or thick as the substantially planar portion. The stop pieces 190 are also not as wide as the substantially planar portion or flange 162. In other words, each stop piece 190 only occupies a section of the side edge of the transverse base 150 and not the entire width of the transverse base, although theoretically a wider stop piece 190 may be incorporated that is about the same width as the transverse base. The top surface of each stop piece 190 can also be offset from the top of the planar portion 162. In some examples, the stop pieces 190 can extend radially outwardly a greater distance than the width of the shield 108 measured between the two exterior surfaces of the sidewalls 110, 112 or can be short of the distance between the two sidewalls. If the stop pieces 190 extend a greater radial distance, they can stop the shield from over-rotation even if the shield spreads or widens, such as distort, upon contacting the stop pieces 190. Once the shield 108 contacts at least one stop piece 190, the base end 116 of each of the sidewalls 110, 112, 114 is prevented from moving past, such as further rotating, beyond an at rest point when the shield 108 is in a protected position in one rotational direction. In the protected position, the shield 108 covers the needle 104 and locks against either the needle 104, the central post or distal cylindrical portion 154, or the needle hub 106, such as the transverse base 150.

In the embodiment of the needle hub 106 shown in Figure 6, the substantially planar portion 162 incorporates two stop pieces 190, each of which comprising a planar surface to abut the shield 108. The stop pieces 190 each includes a flat distal side 192, which is configured to make contact a proximal edge or contact edge 194 of the shield 108. The stop piece shown in Figure 6 includes rounded edges 196 on the proximal side of the stop piece 190. The stop pieces 190 may be placed or positioned along the two side edges 146a of the planar portion or flange 162, such as anywhere between the notch 174 and the socket 142 (FIG. 5). The locations of the distal surfaces 192 of the two stop pieces 190 relative to the flange 162 may also vary depending on where the contact edges 194 of the first and second sidewalls 110, 112 contact the stop pieces 190 during closing rotation of the shield 108. The placement of the stop pieces 190 should also be selected in conjunction with the design and placement of the hooks 130, the tabs 136, and the notches 174 so that collectively they allow locking of the shield 108 to the needle hub 106 without interference. In one example, the stop pieces 190 and the first and second sidewalls 110, 112 of the shield 108 can have latching or locking detents so that when the sidewalls 110, 112 abut the stop pieces 190, the latching or locking detents prevent the shield 108 from separating from the hub 106 and re-open to expose the needle 104.

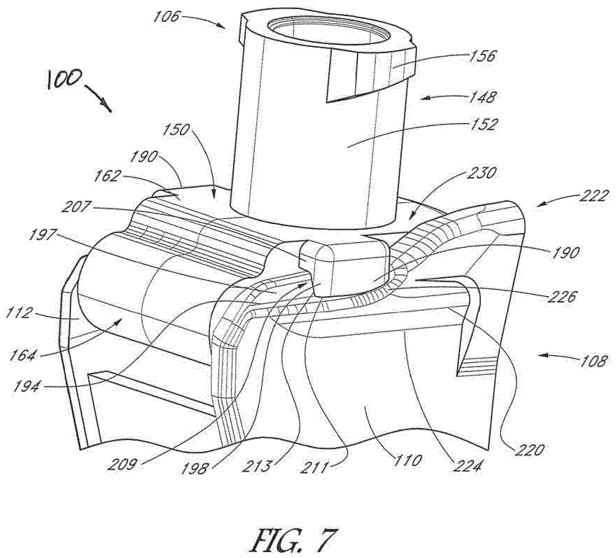

Yet another alternative embodiment of a needle assembly 100 is shown in FIG. 7. The needle assembly 100 has a needle hub 106 and a shield 108 connected to the needle hub in a ball-and-socket arrangement, similar to that shown in FIGs. 1-5. In the present embodiment, the transverse base portion 150 includes two stop pieces 190, on opposite sides of the substantially planar side edges 146a and corresponding to the sides of the two sidewalls 110, 112. The stop pieces 190 in the present embodiment each includes a parallelepiped base, or simply a base, 207, similar to the stop piece of Figure 6. Each base 207 can be viewed as a radially extending member in that it extends radially of the lengthwise axis of the needle. In addition, the stop pieces 190 of the embodiment of Figure 7 each includes a longitudinally extending member, capture element, or capture portion 198. The capture elements or portions 198 extend directly from the two respective bases 207. In one example, the base 207 and the capture portion 198 of each stop piece 190 form a generally "L" shape structure when viewing from an end. In other examples, the base and the capture portion can have other shapes, such as a "T" shape. Each capture portion 198 can be viewed as an axially extending member in that it extends along the same axial direction as the lengthwise axis of the needle. The capture portions 198 on the two stop pieces 190 are sized and shaped to overlap a reduced depth portion 197 of the shield sidewall 110, 112. This reduced depth portion 197 of each sidewall extends transversely, beginning near the hinge component 164 and ending near the middle of the each of the first and second sidewalls 110, 112 and has a dimension that is smaller or less than other depth portions, such as being thinner in dimension compared to other wall surfaces of the same sidewall. The base 207 and the capture portion 198 of each stop piece 190 define a gap or retention space 209 for accommodating the reduced depth portion 197 of the respective sidewall. The two capture portions 198 of the two stop pieces restrict the first and second sidewalls 110, 112 from deflecting radially outwardly away from the lengthwise axis of the needle hub to slip past the two stop pieces and over-rotate. In some examples, only a single stop piece 190 having a base 207 and a capture portion 198 is incorporated with the transverse base portion 150.

The present stop pieces 190 are understood to not only stop the shield 108 from over-rotation by providing radially extending stop surfaces to interfere with the two sidewalls 110, 112 as they rotate, they also prevent or limit outward radial deflection of the two sidewalls 110, 112 by capturing the two sidewalls within retention spaces 209 defined at least in part by the two capture portions 198. In one example, each stop piece comprises a radially extending member and an axially extending member. For example, the base 207 can form a radially extending member and the capture portion 198 can form an axially extending member, both relative to a lengthwise axis of the needle hub or the needle. Thus, aspects of the present disclosure are understood to include at least one of the plurality of sidewalls of the shield 108 contacting the at least one stop piece to prevent over rotation of the shield 108 in one rotational direction when the shield 108 is in a protected position over the needle 104. Another aspect of the present disclosure is understood to include means for limiting outward radial deflection of at least one of the plurality of sidewalls of the shield. The means can include a capture portion 198 extending from a base 207 on a stop piece 190. In other examples, the bases 207 of the stop pieces 190extend sufficiently radially so that the corresponding sidewalls on the shield can be received within the retention spaces 209 without the need for incorporating reduced depth portions 197 on the sidewalls. In some examples, the edge of each sidewall can also be enlarged and the retention spaces 209 are sized and shaped to accommodate the enlarged sections of the sidewalls.

As described above, as a user rotates the shield 108 to a closed position over the needle 104, the tabs 136 (Figure 3) on the shield 108 come into contact with the substantially planar portion 162 of the hub 106. As the tabs 136 come into contact with the substantially planar portion 162, the width of the tabs 136 relative to the width of the planar portion 162 causes the sidewalls 110, 112 on the shield to deflect transversely outwardly, away from the lengthwise axis of the needle and radially of the lengthwise axis of the needle hub 106. This radial deflection of the two sidewalls is greatest at the tabs and decreases as it moves towards the end of the first and second sidewalls 110, 112 with the third sidewall 114. The reduced depth portion 197 of each of the first and second sidewalls slides pass the distal most end 211 of the capture portion 198, between an inside edge 213 of the capture portion 198 and the corresponding side edge 146a of the planar portion 162 transversely opposite the inside edge 213. The process of passing the reduced depth portion 197 between the distal most end 211 of the capture portion 198 between the inside edge 213 of the capture portion 198 and a side of the substantially planar portion 162 effectively captures the respective sidewall 110 or 112 in the retention space 209. Said another way, the capture portion 198 captures the reduced depth portion 197 within the retention space 209 as the shield 108 rotates to close over the needle. In an example, the radially extending portion of each stop piece 190 is sufficiently long or is sized and shaped so that a gap or clearance is provided to ensure sufficient space for the reduced depth portion 197 of the respective sidewall to slide within the retention space 209. In other words, there should be sufficient clearance or tolerance within the retention space 209 to accommodate the respective sidewall of the shield as the sidewall slides inside the retention space.

The contact edge 194 of each sidewall adjacent the reduced depth portion 197 and the distal face 211 of the capture portion 198 of the stop piece can be configured to contact the shield in the shield 108 lock position by providing an increased depth section 220. The increased depth section 220 increases the thickness of each respective sidewall, making the sidewall thicker at and near the base section 222 of the sidewall. The increased depth section 220 starts at a transverse line 224 distal of the proximal edge 194 of the sidewall and transitions from this point away from the sidewall, increasing the thickness such that a rounded proximal edge 226 extends outside of the stop piece 190.

At an end point of the contact edge 194 closest to the hinge component 164, the contact edge 194 is generally parallel to the proximal surface 230 of the substantially planar portion 162. At a halfway point along the contact edge 194, the contact edge surface is still generally parallel to the proximal surface 230 of the substantially planar portion 162. Just past the halfway point in the direction away from the hinge component 164, the contact edge 194 begins to curve, and follows a geometrically proximally curving path. Said another way, as the path of the contact edge 194 moves away from the hinge component 164 and towards the tab 136, the contact edge 194 curves increasingly proximally. In the embodiment shown, the distal face 211 of the capture portion 198 of the stop piece is curved to match the curve of the contact edge 194 along the distal face 211 of the capture portion 198. In other embodiments, the contact edge 194 remains generally parallel to the proximal surface 230 of the substantially planar portion 164 for its entire length, with an angled transition at the opposite end. In these embodiments, the distal face 211 of the capture portion 198 is sized and shaped to match the contact edge, similar to the stop piece 190 embodiment of Figures 5 and 6. In some examples, the entire lower portions of the first and second sidewalls are sufficiently narrow or thin to ensure passing into the retention spaces without the contoured surfaces or with less contoured surfaces described.

As shown in Figures 8 and 9, the shield 108 pivots to cover the needle 104 when a force is applied to the shield, such as by using digital pressure or pushing the shield against a surface to rotate the shield to a closed position. The shield 108 pivots until the hooks 130 snap around the needle 104 and/or the tabs 136 snap into the notches 174 on the needle hub 106. With the needle 104 safely surrounded by the shield 108, the assembly 100 is ready to be discarded. Note that the needle hinged cap embodiment of FIG. 8 incorporates a single hook 130 to hook around the needle 104 and the needle hinge cap assembly of FIG. 9 does not incorporate any hook extending from the central wall. Instead, the shield 108 FIG. 9 is secured to the needle hub 106 by engaging the tabs 136 on the shield with the notches 174 on the needle hub 106. Optionally, a hook 130 may be provided to hook onto the needle 104 and/or to hook onto the central post 280 of the needle hub 106. The stop pieces and the first and second sidewalls can also incorporate locking detents, as previously discussed, to secure the shield to the needle hub.

As further shown in Figures 8 and 9, when the shield 108 reaches the closed position or protected position, the shield 108 is prevented from rotating in the opposite direction, or open direction to re-expose the needle, by the engagement of the tabs 136 with the notches 174 and/or by the hook hooking onto the needle or the post. As shown in Figures 6 and 7, in the same closed position or in the process of closing the shield over the needle, the shield 108 is prevented from over rotating by the contact between the stop pieces 190 and the contact edges 194 of the shield 108. When incorporated, one or more stop pieces can be used to prevent the shield from over-rotating. By preventing rotation in either direction when the shield 108 is in the locked position or protected position shown in FIGs. 6 and 7 and elsewhere, the hinged shield assemblies 100 of the present disclosure prevent or deter accidental or intentional reopening of the shield 108.

## Claims

1. A hinged shield assembly (100) comprising:
a needle hub (106) including a flange (162) and a first hinge part (500) formed with the flange (162), a needle (104) extending from the needle hub (106) and comprising a lengthwise axis;
a shield (108) including a second hinge part (502) that engages the first hinge part (500) to pivotably secure the shield (108) to the hub, the shield (108) further including a plurality of sidewalls (110, 112, 114) that partially surround the needle (104) when the shield (108) is in a protected position; wherein each of the sidewalls (110, 112, 114) comprises a base end (116);
**characterized in that**
the hinged shield assembly (100) further comprises at least one stop piece (190) extending radially of the lengthwise axis from the flange (162) of the needle hub (106) and located in a rotational path of the shield (108) such that at least one of the plurality of sidewalls (110, 112, 114) contacts the at least one stop piece (190) to prevent over rotation of the base end (116) of each of the sidewalls (110, 112, 114) beyond an at rest point upon contact with the at least one stop piece (190) in one rotational direction when the shield (108) is in the protected position, and wherein the at least one stop piece (190) includes a flat distal side configured to contact a proximal edge or contact edge of the at least one of the plurality of sidewalls (110, 112, 114) of the shield (108).

2. The hinged shield assembly (100) of Claim 1, wherein the at least one stop piece (190) comprises a base (207) having a radially extending planar surface to abut the shield (108).

3. The hinged shield assembly (100) of Claim 2, further comprising a capture element (198) extending from the base (207) in an axial direction to define a retention space (209).

4. The hinged shield assembly (100) of any of the preceding claims, further comprising a reduced depth portion (197) formed on at least one of the plurality of sidewalls (110, 112, 114) on a side of the hinged shield assembly (100) corresponding to the at least one stop piece (190).

5. The hinged shield assembly (100) of any of the preceding claims, wherein the first hinge part (500) comprises sockets (142) and the second hinge part (502) comprises balls (134).

6. The hinged shield assembly (100) of any of claims 2 to 5, further comprising a second stop piece (190) comprising a base (207) and wherein the two bases (207) define a stop width.

7. The hinged shield assembly (100) of claim 6, wherein a first sidewall (110) and a second sidewall (112) of the plurality of sidewalls (110, 112, 114) define a shield width and wherein the stop width is wider than the shield width or is equal to the shield width.

8. The hinged shield assembly (100) of any of claims 2 to 7, wherein the at least one stop piece (190) is located on a transverse base (150) defining the flange (162), which intersects an elongated portion (148) on the needle hub (106) between a distal cylindrical portion (154) and a proximal cylindrical portion (152).

9. The hinged shield assembly (100) of claim 8, wherein the flange (162) has a first edge, a second edge, and a first side edge (146a) and wherein the at least one stop piece (190) is located on the side edge (146a), between the first edge and the second edge.

10. The hinged shield assembly (100) of claim 9, wherein the flange (162) has an upper surface facing the distal cylindrical portion (154), and wherein the radially extending planar surface of the base (207) is offset from the upper surface.

11. The hinged shield assembly (100) of any of claims 3 to 10, further comprising a second base (207) and a second capture element (198) and wherein the two bases (207) define radially extending members and the two capture elements (198) define axially extending members.

12. A method of manufacturing a hinged shield assembly (100) comprising:
forming a needle hub (106) including a flange (162) and a first hinge part (500) formed with the flange;
extending a needle (104) from the needle hub (106), said needle (104) comprising a lengthwise axis;
forming a shield (108) including a second hinge part (502) that engages the first hinge part (500) to pivotably secure the shield (108) to the hub, the shield (108) further including a plurality of sidewalls (110, 112, 114) that partially surround the needle (104) when the shield (108) is in a protected position; wherein each of the sidewalls (110, 112, 114) comprises a base end (116); **characterized in that** the method comprises
forming two stop pieces (190) extending from the flange (162) radially of the lengthwise axis and positioning the two stop pieces (190) in a rotational path of the shield (108) such that a proximal edge or contact edge of a first sidewall (110) and a proximal edge or contact edge of a second sidewall (112) of the plurality of walls (110, 112, 114) contact a flat distal side of the two stop pieces (190) to prevent over rotation of the base end (116) of the first and second sidewalls (110, 112) beyond an at rest point upon contact with the two stop pieces (190) in one rotational direction when the shield (108) is in the protected position.

13. The method of Claim 12, wherein each stop piece (190) has a base (207) and a capture element (198) extending from the base (207) in an axial direction to define a retention space (209).

14. The method of Claim 13, wherein at least a portion of the base end (116) of the first sidewall (110) and at least a portion of the base end (116) of the second sidewall (112) are both located in the two retention spaces (209).

15. The method of Claim 14, wherein the base end (116) of the first sidewall (110) and the base end (116) of the second sidewall (112) each comprises a reduced depth portion (197) and wherein the reduced depth portion (197) is located in a respective retention space (209).

## Patentansprüche

1. Schildanordnung mit Scharnier (100) umfassend:
einen Nadelansatz (106) mit einem Flansch (162) und einem ersten Scharnierteil (500), das mit dem Flansch (162) ausgebildet ist, wobei sich eine Nadel (104) von dem Nadelansatz (106) erstreckt und eine Längsachse aufweist;
ein Schild (108) mit einem zweiten Scharnierteil (502), das mit dem ersten Scharnierteil (500) in Eingriff tritt, um das Schild (108) schwenkbar an dem Ansatz zu befestigen, wobei das Schild (108) weiterhin eine Vielzahl von Seitenwänden (110, 112, 114) aufweist, die die Nadel (104) teilweise umgeben, wenn sich das Schild (108) in einer Schutzposition befindet; wobei jede der Seitenwände (110, 112, 114) ein Basisende (116) aufweist;
**dadurch gekennzeichnet, dass**
die Schildanordnung mit Scharnier (100) weiterhin wenigstens ein Anschlagstück (190) umfasst, das sich radial zu der Längsachse von dem Flansch (162) des Nadelansatzes (106) erstreckt und sich in einem Drehweg des Schildes (108) derart befindet, dass wenigstens eine der Vielzahl von Seitenwänden (110, 112, 114) mit dem wenigstens einem Anschlagstück (190) in Eingriff tritt, um eine Überdrehung des Basisendes (116) eines jeden der Seitenwände (110, 112, 114) über einen Ruhepunkt hinaus bei Kontakt mit dem wenigstens einen Anschlagstück (190) in einer Drehrichtung zu verhindern, wenn sich das Schild (108) in der Schutzposition befindet, und wobei das wenigstens eine Anschlagstück (190) eine flache distale Seite aufweist, die derart konfiguriert ist, um mit einer proximalen Kante oder einer Kontaktkante der wenigstens einen der Vielzahl von Seitenwänden (110, 112, 114) des Schildes (108) in Eingriff zu treten.

2. Schildanordnung mit Scharnier (100) nach Anspruch 1, wobei das wenigstens eine Anschlagstück (190) eine Basis (207) mit einer sich radial erstreckenden, ebenen Oberfläche aufweist, um auf das Schild (108) anzuliegen.

3. Schildanordnung mit Scharnier (100) nach Anspruch 2 weiterhin umfassend ein Fangelement (198), das sich von der Basis (207) in einer axialen Richtung erstreckt, um einen Rückhalteraum (209) zu definieren.

4. Schildanordnung mit Scharnier (100) nach einem der vorhergehenden Ansprüche weiterhin umfassend einen Abschnitt mit reduzierter Tiefe (197), der an wenigstens einer der Vielzahl von Seitenwände (110, 112, 114) auf einer Seite der Schildanordnung mit Scharnier (100) ausgebildet ist, die dem wenigstens einen Anschlagstück (190) entspricht.

5. Schildanordnung mit Scharnier (100) nach einem der vorhergehenden Ansprüche, wobei das erste Scharnierteil (500) Sockel (142) und das zweite Scharnierteil (502) Kugeln (134) umfasst.

6. Schildanordnung mit Scharnier (100) nach einem der Ansprüche 2 bis 5, weiterhin umfassend ein zweites Anschlagstück (190), das eine Basis (207) aufweist, und wobei die beiden Basen (207) eine Anschlagbreite definieren.

7. Schildanordnung mit Scharnier (100) nach Anspruch 6, wobei eine erste Seitenwand (110) und eine zweite Seitenwand (112) der Vielzahl von Seitenwänden (110, 112, 114) eine Schildbreite definieren, und wobei die Anschlagbreite breiter oder gleich der Schildbreite ist.

8. Schildanordnung mit Scharnier (100) nach einem der Ansprüche 2 bis 7, wobei sich das wenigstens eine Anschlagstück (190) an einer quer verlaufenden Basis (150) befindet, die den Flansch (162) definiert, der einen länglichen Abschnitt (148) an dem Nadelansatz (106) zwischen einem distalen zylindrischen Abschnitt (154) und einem proximalen zylindrischen Abschnitt (152) schneidet.

9. Schildanordnung mit Scharnier (100) nach Anspruch 8, wobei der Flansch (162) eine erste Kante, eine zweite Kante und eine erste Seitenkante (146a) aufweist und wobei sich das wenigstens eine Anschlagstück (190) an der Seitenkante (146a) zwischen der ersten Kante und der zweiten Kante befindet.

10. Schildanordnung mit Scharnier (100) nach Anspruch 9, wobei der Flansch (162) eine obere Oberfläche aufweist, die dem distalen zylindrischen Abschnitt (154) zugewandt ist, und wobei die sich radial erstreckende, ebene Oberfläche der Basis (207) von der oberen Oberfläche versetzt angeordnet ist.

11. Klappbare Schildanordnung mit Scharnier (100) nach einem der Ansprüche 3 bis 10, die ferner eine zweite Basis (207) und ein zweites Fangelement (198) umfasst, und wobei die beiden Basen (207) sich radial erstreckende Elemente definieren und die beiden Fangelemente (198) sich axial erstreckende Elemente definieren.

12. Verfahren zum Herstellen einer Schildanordnung mit Scharnier (100) umfassend:
das Ausbilden eines Nadelansatzes (106) mit einem Flansch (162) und einem ersten Scharnierteil (500), das mit dem Flansch ausgebildet ist;
das Verlängern einer Nadel (104) von dem Nadelansatz (106), wobei die Nadel (104) eine Längsachse aufweist;
das Ausbilden eines Schildes (108) mit einem zweiten Scharnierteil (502), das mit dem ersten Scharnierteil (500) in Eingriff tritt, um das Schild (108) schwenkbar an dem Ansatz zu befestigen, wobei das Schild (108) weiterhin eine Vielzahl von Seitenwänden (110, 112, 114) aufweist, die die Nadel (104) teilweise umgeben, wenn sich das Schild (108) in einer Schutzposition befindet; wobei jede der Seitenwände (110, 112, 114) ein Basisende (116) aufweist; **dadurch gekennzeichnet, dass** das Verfahren umfasst:
das Ausbilden von zwei Anschlagstücken (190), die sich von dem Flansch (162) radial zu der Längsachse erstrecken, und
das Positionieren der beiden Anschlagstücke (190) in einem Drehweg des Schildes (108) derart, dass eine proximale Kante oder Kontaktkante einer ersten Seitenwand (110) und eine proximale Kante oder Kontaktkante einer zweiten Seitenwand (112) der Vielzahl von Wänden (110, 112, 114) mit einer flachen, distalen Seite der beiden Anschlagstücke (190) in Eingriff tritt, um eine Überdrehung des Basisendes (116) der ersten und zweiten Seitenwand (110, 112) über einen Ruhepunkt hinaus bei Kontakt mit den beiden Anschlagstücken (190) in einer Drehrichtung zu verhindern, wenn sich das Schild (108) in der Schutzposition befindet.

13. Verfahren nach Anspruch 12, wobei jedes Anschlagstück (190) eine Basis (207) und ein Fangelement (198) aufweist, das sich von der Basis (207) in einer axialen Richtung erstreckt, um einen Rückhalteraum (209) zu definieren.

14. Verfahren nach Anspruch 13, wobei sich wenigstens ein Abschnitt des Basisendes (116) der ersten Seitenwand (110) und wenigstens ein Abschnitt des Basisendes (116) der zweiten Seitenwand (112) in den beiden Rückhalteräumen (209) befinden.

15. Verfahren nach Anspruch 14, wobei das Basisende (116) der ersten Seitenwand (110) und das Basisende (116) der zweiten Seitenwand (112) jeweils einen Abschnitt mit reduzierter Tiefe (197) umfassen und wobei sich der Abschnitt mit reduzierter Tiefe (197) in einem jeweiligen Rückhalteraum (209) befindet.

## Revendications

1. Ensemble d'étui à charnières (100) comprenant :
un raccord d'aiguille (106) comportant une bride (162) et une première partie charnière (500) formée avec la bride (162), une aiguille (104) s'étendant à partir du raccord d'aiguille (106) et comprenant un axe longitudinal ;
un étui (108) comportant une seconde partie charnière (502) qui vient en prise avec la première partie charnière (500) pour fixer de manière pivotante l'étui (108) au raccord, l'étui (108) comportant en outre une pluralité de parois latérales (110, 112, 114) qui entourent partiellement l'aiguille (104) lorsque l'étui (108) est dans une position protégée ; dans lequel chacune des parois latérales (110, 112, 114) comprend une extrémité de base (116) ;
**caractérisé en ce que**
l'ensemble d'étui à charnières (100) comprend en outre au moins une pièce d'arrêt (190) s'étendant radialement par rapport à l'axe longitudinal à partir de la bride (162) du raccord d'aiguille (106) et située dans un trajet de rotation de l'étui (108) de telle sorte qu'au moins l'une de la pluralité de parois latérales (110, 112, 114) entre en contact avec l'au moins une pièce d'arrêt (190) pour empêcher une rotation excessive de l'extrémité de base (116) de chacune des parois latérales (110, 112, 114) au-delà d'un point de repos lors du contact avec l'au moins une pièce d'arrêt (190) dans un sens de rotation lorsque l'étui (108) est dans la position protégée, et dans lequel l'au moins une pièce d'arrêt (190) comporte un côté distal plat conçu pour entrer en contact avec un bord proximal ou un bord de contact de l'au moins une de la pluralité de parois latérales (110, 112, 114) de l'étui (108).

2. Ensemble d'étui à charnières (100) selon la revendication 1, dans lequel l'au moins une pièce d'arrêt (190) comprend une base (207) ayant une surface plane s'étendant radialement pour venir en butée contre l'étui (108).

3. Ensemble d'étui à charnières (100) selon la revendication 2, comprenant en outre un élément de capture (198) s'étendant à partir de la base (207) dans une direction axiale pour définir un espace de rétention (209).

4. Ensemble d'étui à charnières (100) selon l'une quelconque des revendications précédentes, comprenant en outre une partie de profondeur réduite (197) formée sur au moins l'une de la pluralité de parois latérales (110, 112, 114) sur un côté de l'ensemble d'étui à charnières (100) correspondant à l'au moins une pièce d'arrêt (190).

5. Ensemble d'étui à charnières (100) selon l'une quelconque des revendications précédentes, dans lequel la première partie charnière (500) comprend des douilles (142) et la seconde partie charnière (502) comprend des billes (134).

6. Ensemble d'étui à charnières (100) selon l'une quelconque des revendications 2 à 5, comprenant en outre une seconde pièce d'arrêt (190) comprenant une base (207) et dans lequel les deux bases (207) définissent une largeur d'arrêt.

7. Ensemble d'étui à charnières (100) selon la revendication 6, dans lequel une première paroi latérale (110) et une seconde paroi latérale (112) de la pluralité de parois latérales (110, 112, 114) définissent une largeur d'étui et dans lequel la largeur d'arrêt est plus large que la largeur d'étui ou est égale à la largeur d'étui.

8. Ensemble d'étui à charnières (100) selon l'une quelconque des revendications 2 à 7, dans lequel l'au moins une pièce d'arrêt (190) est située sur une base transversale (150) définissant la bride (162), qui coupe une partie allongée (148) sur le raccord d'aiguille (106) entre une partie cylindrique distale (154) et une partie cylindrique proximale (152).

9. Ensemble d'étui à charnières (100) selon la revendication 8, dans lequel la bride (162) a un premier bord, un second bord et un premier bord latéral (146a) et dans lequel l'au moins une pièce d'arrêt (190) est située sur le bord latéral (146a), entre le premier bord et le second bord.

10. Ensemble d'étui à charnières (100) selon la revendication 9, dans lequel la bride (162) a une surface supérieure faisant face à la partie cylindrique distale (154), et dans lequel la surface plane s'étendant radialement de la base (207) est décalée de la surface supérieure.

11. Ensemble d'étui à charnières (100) selon l'une quelconque des revendications 3 à 10 comprenant en outre une seconde base (207) et un second élément de capture (198) et dans lequel les deux bases (207) définissent des organes s'étendant radialement et les deux éléments de capture (198) définissent des organes s'étendant axialement.

12. Procédé de fabrication d'un ensemble d'étui à charnières (100), comprenant :
la formation d'un raccord d'aiguille (106) comportant une bride (162) et une première partie charnière (500) formée avec la bride ;
l'extension d'une aiguille (104) à partir du raccord d'aiguille (106), ladite aiguille (104) comprenant un axe longitudinal ;
la formation d'un étui (108) comportant une seconde partie charnière (502) qui vient en prise avec la première partie charnière (500) pour fixer de manière pivotante l'étui (108) au raccord, l'étui (108) comportant en outre une pluralité de parois latérales (110, 112, 114) qui entourent partiellement l'aiguille (104) lorsque l'étui (108) est dans une position protégée ; dans lequel chacune des parois latérales (110, 112, 114) comprend une extrémité de base (116) ;
**caractérisé en ce que** le procédé comprend
la formation de deux pièces d'arrêt (190) s'étendant à partir de la bride (162) radialement par rapport à l'axe longitudinal et le positionnement des deux pièces d'arrêt (190) dans un trajet de rotation de l'étui (108) de telle sorte qu'un bord proximal ou bord de contact d'une première paroi latérale (110) et qu'un bord proximal ou bord de contact d'une seconde paroi latérale (112) de la pluralité de parois (110, 112, 114) entre en contact avec un côté distal plat des deux pièces d'arrêt (190) pour empêcher une rotation excessive de l'extrémité de base (116) des première et seconde parois latérales (110, 112) au-delà d'un point de repos lors du contact avec les deux pièces d'arrêt (190) dans un sens de rotation lorsque l'étui (108) est dans la position protégée.

13. Procédé selon la revendication 12, dans lequel chaque pièce d'arrêt (190) a une base (207) et un élément de capture (198) s'étendant à partir de la base (207) dans une direction axiale pour définir un espace de rétention (209).

14. Procédé selon la revendication 13, dans lequel au moins une partie de l'extrémité de base (116) de la première paroi latérale (110) et au moins une partie de l'extrémité de base (116) de la seconde paroi latérale (112) sont toutes deux situées dans les deux espaces de rétention (209).

15. Procédé selon la revendication 14, dans lequel l'extrémité de base (116) de la première paroi latérale (110) et l'extrémité de base (116) de la seconde paroi latérale (112) comprennent chacune une partie de profondeur réduite (197) et dans lequel la partie de profondeur réduite (197) est située dans un espace de rétention (209) respectif.
